# EUROPEAN PATENT APPLICATION

(11) **EP 4 635 405 A1**
(43) Date of publication of application: **22.10.2025**
(21) Application number: 25170221.3
(22) Date of filing: 11.04.2025
(51) Int. Cl.: A61B 5/00, A61B 5/145, A61B 5/1459, A61B 5/1473, G01N 21/00, G01N 21/77

(54) **DEVICE FOR DETECTING THE CONCENTRATION OF A DRUG**

(30) Priority: 15.04.2024 IT 202400008506
(71) Applicant: Università di Pisa, 56126 Pisa (IT)
(72) Inventor: Barillaro, Giuseppe, 56121 Pisa (PI) (IT); Corsi, Martina, 57124 Livorno (LI) (IT); Maurina, Elena, 05100 Terni (TR) (IT)
(74) Representative: Pretato, Filippo

(57) **Abstract**

A device for detecting the concentration of a drug, wherein the drug comprises at least one functional group suited to emit at least one beam of electromagnetic radiation, such device comprises at least one main body provided with a binding surface intended to intercept the drug to be detected. The main body is provided with a porous structure defined by a plurality of pores that delimit the binding surface within them. The main body comprises at least one receptor linked to the binding surface and configured to chemically bind the drug in the plurality of pores. The intensity of the beam of electromagnetic radiation emitted by the drug bound in the plurality of pores is proportional to the concentration of the drug.

## Description

### Field of application of the invention

The present invention relates to a device for detecting the concentration of a drug in a patient. In particular, the device in question is configured to be implanted under the skin of a patient to detect the concentration of a predetermined drug used during a treatment.

Therefore, the present invention finds advantageous use in the technical field of the production and marketing of sensors, apparatus or other devices for the detection and control of drugs.

### State of the art

There has long been a need to monitor the concentration of drugs administered to a patient, particularly in the case of drugs that, at high concentrations, can be harmful to the patient's body.

For example, in the case of antitumor drugs (such as, for example, the case of chemotherapy drugs or similar), it is necessary to constantly monitor the concentration of the drug itself to avoid intoxication of the patient, which could lead to numerous side effects.

For this purpose, common practice is to examine a sample of the patient's blood in order to detect the concentration of the drug of interest.

Obviously, this procedure is long, expensive and is not able to constantly and/or continuously detect the trend of the drug concentration, especially during treatment.

In recent years, the technical sector of reference has seen the birth of appropriate sensors capable of detecting some biochemical parameters of the patient.

In particular, in the last decade there have been significant progress in the development and production of sensors capable of detecting specific molecules, called in jargon "target" molecules.

Traditional sensors are generally used in medical clinics authorized to process biological samples, such as blood and/or urine.

The main drawback of known sensors, however, is that they detect the presence and/or quantity of a specific target molecule at a single, precise moment in time.

This prevents their use for the continuous and real-time monitoring of desired drugs.

In recent years, sensors configured to continuously detect one or more target molecules have been developed. In particular, these sensors are configured to be implanted subcutaneously to monitor the aforementioned molecules, such as glucose in patients with diabetes.

Sensors are known in the technical field of reference suited to detect in a continuous and real-time manner complex target molecules, such as chemotherapeutic drugs.

For example, sensors are known comprising microelectrodes of nanoporous gold, chemically functionalized with an aptamer functionalized with methylene blue (MB), which is configured to chemically bind with a chemotherapeutic drug (in particular doxorubicin).

The microelectrodes detect an increase in electron transfer with the aptamer functionalized with methylene blue, wherein this increase is proportional to the drug concentration.

In this way, the known type sensor allows to detect the concentration of the chemotherapeutic drug, transducing the value of the increase in electron transfer into a value of the concentration of the drug bound to the aptamer functionalized with methylene blue.

The main drawback of these known sensors is that they must be implanted and then surgically removed. Obviously, this requirement translates into an extremely high cost of use and management of the sensor.

Another drawback is that the use of the sensor is extremely invasive for the patient who often already suffers from previous pathologies.

### Presentation of the invention

The purpose of the present invention is to overcome and remedy the drawbacks of the above-mentioned prior art by providing a device for detecting the concentration of a drug.

In particular, the purpose of the present invention is to provide a device for detecting the concentration of a drug that allows the concentration of at least one drug to be detected in a constant and/or continuous manner.

A further purpose of the present invention is to provide a device for detecting the concentration of a drug that is less invasive for the patient than the sensors of the prior art.

A further purpose of the present invention is to provide a device for detecting the concentration of a drug that is continuously readable even when implanted under the patient's skin.

A further purpose of the present invention is to provide a device for detecting the concentration of a drug that helps to determine in real time the correct dosage of the drug, specific for each patient (known in jargon with the term "fine tuning"), during a cycle of pharmacological treatments.

A further purpose of the present invention is to provide a device for detecting the concentration of a drug that, by helping to obtain a calibration of the dosage of the drug to be administered to the patient, allows to minimize the side effects associated with the drug itself.

A further purpose of the present invention is to provide a device for detecting the concentration of a purpose that is biocompatible with a patient's organism.

A further purpose of the present invention is to provide a device for detecting the concentration of a drug that can be manufactured industrially.

A further purpose of the present invention is to provide a device for detecting the concentration of a drug that is economically advantageous and profitable.

A further purpose of the present invention is to provide a device for detecting the concentration of a drug that is structurally and functionally completely reliable.

A further object of the present invention is to provide a device for detecting the concentration of a drug that has an improved and/or alternative character compared to the solutions of the prior art.

These objects, together with others that will be better clarified below, are achieved by a device for detecting the concentration of a drug in accordance with claim 1.

Further objects, which will be better described below, are achieved by a device for detecting the concentration of a drug in accordance with the dependent claims.

According to a further aspect of the present invention, an assembly for detecting the concentration of a drug is provided, of the type in accordance with claim no. 14.

### Brief description of the drawings

The advantages and features of the present invention will be clearly apparent from the following detailed description of some preferred but non-limiting configurations of a drug concentration detection device with particular reference to the following drawings:
- Figure 1 shows a schematic perspective view of a device for detecting the concentration of a drug, which is the subject of the present invention;
- Figure 2 shows a schematic front view of an example of use on a patient of the detection device according to the invention;
- Figure 3 and Figure 4 show a sectional view of a main body of the detection device according to the invention;
- Figure 5 shows a top view of a detection device according to the invention and an enlargement thereof;
- Figure 6 shows a schematic perspective view of a first sub-step of a step of preparation of the method of manufacturing the detection device, which is also the subject of the present invention;
- Figure 7 shows a further schematic perspective view of a second sub-step of the step of preparation of the method of manufacturing the detection device, which is also the subject of the present invention;
- Figure 8 shows a further schematic perspective view of a third sub-step of the preparation step of the method of manufacturing the detection device, which is also the subject of the present invention;
- Figure 9 shows a schematic perspective view of a semi-finished product obtained following the preparation step according to the method object of the invention;
- Figure 10 shows a schematic view of a first sub-step of the treatment step of the internal surface of the pores of the porous silica layer of the method according to the invention;
- Figure 11 shows a schematic view of a second sub-step of the treatment step of the internal surface of the pores of the porous silica layer of the method according to the invention;
- Figure 12 shows a schematic view of a third sub-step of the treatment step of the internal surface of the pores of the porous silica layer of the method according to the invention;
- Figure 13 shows a schematic view of the operation of the device according to the invention, in which the connection of the detection device with the drug to be detected is visible;
- Figure 14 shows a graphic concerning the intensity of fluorescence detected as the concentration of the drug varies.

### Detailed description of the invention

The present invention relates to a device for detecting the concentration of a drug F.

The device, which is the subject of the present invention, hereinafter indicated with the reference number 1, may be mainly used to identify and detect the presence and concentration of a drug, preferably present in a patient.

Therefore, the device 1 according to the invention finds advantageous use in the technical sector of the production and marketing of diagnostic apparatus and devices.

Advantageously, the device 1 in question can be used to directly detect from the body P of a patient the presence and/or concentration of a specific drug, in particular (but not limited to) an antitumor drug.

Hereinafter, the term "concentration" is to be intended as a mass of drug F present in a certain volume of liquid, for example, of the patient's plasma.

The device 1 of the present invention is intended to detect the concentration of a drug F, which comprises at least one functional group suited to emit at least one beam R1 of electromagnetic radiation.

The term "electromagnetic radiation" must be understood, pursuant to the present patent application, to mean any emission of electromagnetic waves, at any wavelength.

Preferably, the drug F detectable by the device 1 according to the invention is a drug suited to emit a beam R1 of electromagnetic waves by means of photoluminescence, in particular fluorescence.

Preferably, the beam R1 of electromagnetic waves emitted by the drug F has a wavelength between 500nm and 650 nm, and in particular between 540 nm and 590 nm. More specifically, the beam R1 of electromagnetic waves has a greater intensity at a first wavelength of approximately 540 nm and at a second wavelength of approximately 590 nm.

The device 1, object of the present invention, comprises at least one main body 2.

According to a preferred embodiment of the present invention, said main body 2 has a laminar shape and extends on a development plane X.

Preferably, said main body of laminar shape has a thickness between 2 µm and 10µm, more preferably equal to approximately 5µm.

Said main body 2 is provided with a binding surface 3, intended to intercept said drug F to be detected.

More clearly, the binding surface 3 is configured to receive and chemically bind the drug F to be detected, as described in detail below.

The main body 2 is provided with a porous structure defined by a plurality of pores 5.

Preferably, the main body 2 may comprise one or more laminar layers (porous). As will be clear below, the main body 2 may comprise at least one porous laminar layer 151 and/or one porous extremal layer 154.

Advantageously, the porous laminar layer 151 and/or the extremal layer 154 may be defined by pores 5,5' as described in detail below.

In more detail, each pore 5 of said plurality of pores 5 internally delimits at least partially said binding surface 3.

According to an embodiment of the present invention, each pore 5 of said plurality of pores 5 can have an average diameter preferably between 10 and 50 nm.

Preferably, each pore 5 of said plurality of pores 5 has a diameter equal to approximately 30 nm.

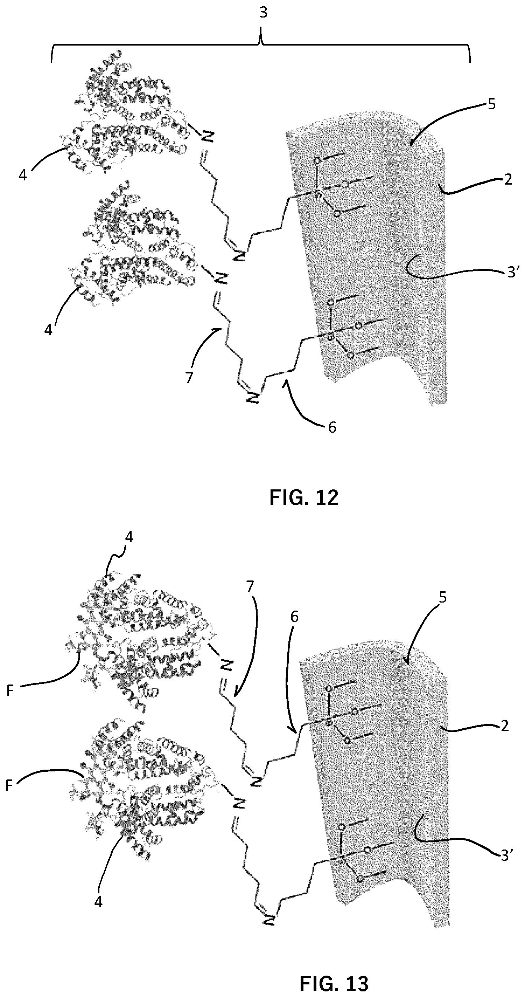

The binding surface 3 comprises at least one receptor 4 configured to chemically bind said drug F in said plurality of pores 5 (as visible in Figure 13).

In other words, the receptor 4 can be anchored to the binding surface 3 to chemically bind said drug F in said plurality of pores 5.

For the purposes of the present description, the term "anchored" (or anchoring) means that a molecule, such as a receptor, is retained on a surface or support through physical interactions or chemical bonds.

According to the invention, said intensity of said beam R1 of electromagnetic radiation emitted by said drug F bound in said plurality of pores 5 is proportional to said concentration of said drug F.

In this way, the device 1 according to the invention allows to generate electromagnetic radiation with intensity directly proportional to the concentration of drug F.

Conveniently, the electromagnetic radiation generated by the device 1 according to the invention is easily detectable from the outside by an apparatus 100 provided with suitable sensor means, as described in detail below.

Preferably, the receptor 4 of said binding surface 3 comprises human serum albumin, which is known in the technical jargon of the sector with the acronym HSA.

Preferably, the receptor 4 is chemically bound to an internal surface 3' of each pore 5 of the main body 2 by at least one linking group 10.

In other words, preferably, the binding surface 3 comprises the internal surface 3' of each pore 5 of said plurality of pores 5, the linking group 10 and the receptor 4.

Preferably, the linking group 10 comprises at least one functionalizing agent 6 and, preferably, at least one coupling agent 7.

The functionalizing agent 6 (and the coupling agent 7) is chemically interposed between the receptor 4 and the binding surface 3.

Advantageously, the 3' internal surface of each pore 5 is functionalized with the functionalizing agent 6 (and with the coupling agent 7) to allow chemical bonding with the receptor 4.

Preferably, the chemical bond between the internal surface 3' of each pore 5 and the receptor 4 is a chemical bond of covalent type.

Preferably, the functionalizing agent 6 is selected from the silane family. More preferably, the functionalizing agent 6 comprises an aminosilane and, in particular, (3-aminopropyl)triethoxysilane, known in jargon with the acronym APTES.

Advantageously, the coupling agent 7 chemically binds the functionalizing agent 6 to the receptor 4.

More in detail, the coupling agent 7 is selected from the aliphatic dialdehyde family and, in particular, comprises glutaraldehyde, known in jargon with the acronym GA.

In other words, the internal surface 3' of each pore 5 has a functionalized layer, defined by the aforementioned linking group 10, which comprises the aforementioned functionalizing agent 6 and the aforementioned coupling agent 7.

Advantageously, at least a portion of said drug F is selected from the anthracycline family. Preferably, the drug F comprises an antineoplastic antibiotic.

As anticipated above, the drug F comprises at least one functional group capable of generating a beam R1 of electromagnetic radiation, for example by photoluminescence.

In accordance with the preferred embodiment of the present invention, the drug F is doxorubicin and, preferably, comprises at least one fluorophore group suited to generate the beam R1 of electromagnetic radiation.

Advantageously, each pore 5 of said plurality of pores 5 has an average diameter between 10 and 50 nm.

Preferably, each pore 5 of said plurality of pores 5 has an average diameter equal to approximately 30 nm.

Advantageously, said binding surface 3 is between 50 and 500 m²/cm³ and preferably approximately equal to 100 m²/cm³.

Preferably, said main body 2 has porosity greater than or equal to 60% and even more preferably greater than or equal to 77%.

Advantageously, said main body 2 is intended to be implanted in the body P of a patient and is made of bioresorbable material.

Conveniently, the bioresorbable material used to make the main body 2 may preferably have null intrinsic fluorescence.

In other words, the bioresorbable material having null intrinsic fluorescence avoids the risk of interfering with the detection of the concentration of the drug flow F. More specifically, the bioresorbable material is not capable of emitting electromagnetic radiation, for example by photoluminescence, which could interfere with the beam R1 of electromagnetic radiation generated by the functional group of the drug F thus altering the detection of the concentration of the drug F itself.

Preferably, the bioresorbable material used to make the main body 2 may preferably be an electrically insulating material.

Preferably, the bioresorbable material used to make the main body 2 may be transparent.

More specifically, the main body 2 may preferably be made of porous silicon oxide (also known as silica), in particular known by the acronym PSiO₂.

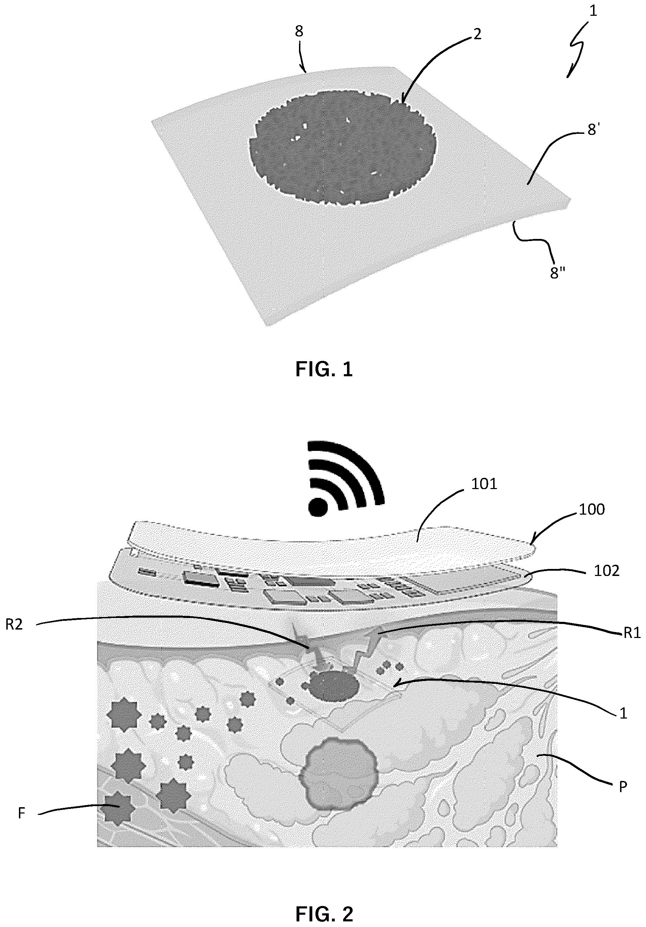

Advantageously, the main body 2 of the device 1 is intended to be implanted inside a body P of a patient. In particular, the main body 2 of the device 1 is intended to be implanted under the skin of the patient, in accordance with the application example illustrated in the attached Figure 2.

Operationally, the main body 2 is intended to degrade upon contact with the subcutaneous chemical environment of the body P of the patient.

More clearly, the main body 2 is intended to be absorbed by the patient's organism approximately 3-5 days after implantation.

In this way, the main body 2 is not invasive and does not require any surgical removal following its use.

Preferably, the device 1 comprises a support element 8 which carries the main body 2 mounted on it.

Conveniently, the support element 8 has a substantially laminar shape and is configured to improve the mechanical properties of the main body 2.

Preferably, the support element 8 is made of flexible material and preferably of polymeric material.

Preferably, the support element 8 is made of bioresorbable material.

More specifically, the support element 8 is made of poly(lactic-co-glycolic acid), known in technical jargon by the acronym PLGA.

Preferably, the support element 8 is provided with a first surface 8' mechanically constrained to said main body 2 and a second surface 8" free, and substantially opposite to the first surface 8'.

Advantageously, the main body 2 is mechanically constrained to the first surface 8' of the support element 8 by heat welding.

More in detail, the polymeric material of the support element 8 grips the plurality of pores 5 (and therefore the porous structure) of the main body 2.

Operationally, the support element 8 allows for safer and easier movement and implantation of the main body 2.

Preferably, each pore 5 of said plurality of pores 5 extends along a development axis Y substantially orthogonal to said development plane X.

More in detail, each pore 5 of said plurality of pores 5 has a substantially elongated shape and extends mainly transversely with respect to the planar development of the main body 2.

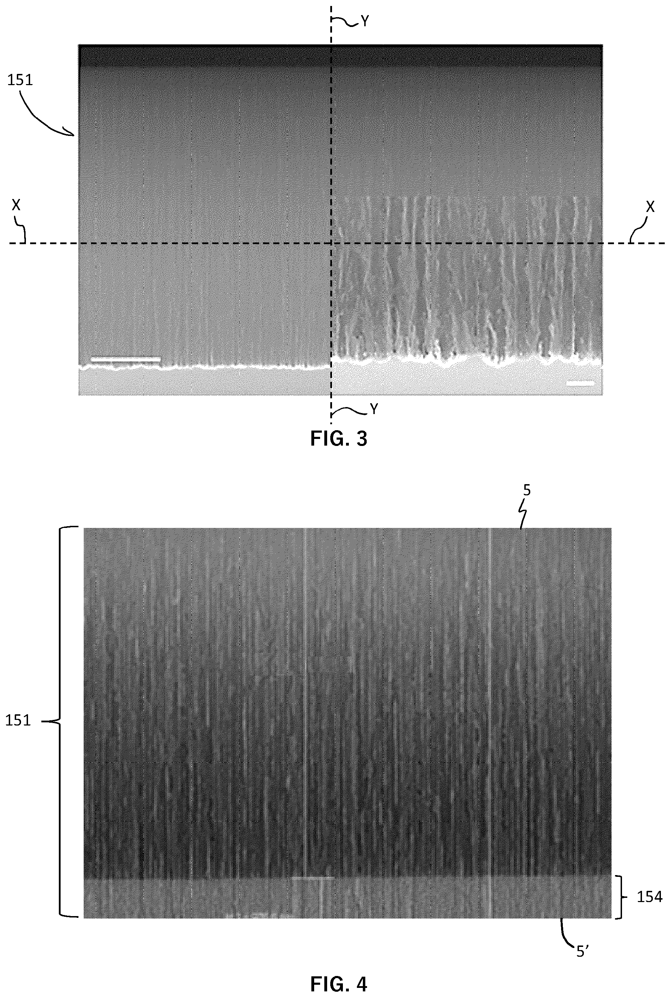

With particular reference to the attached Figure 3, the pores 5 preferably develop substantially parallel to each other. In particular, the pores 5 preferably develop substantially parallel to the axis Y and transversely to the development plane X.

Preferably, the plurality of pores 5 is made by electrochemical etching.

Advantageously, each pore 5 of said plurality of pores 5 has an elongated shape extending along and/or parallel to said axis Y.

Conveniently, the porous structure of the main body 2 preferably comprises at least one porous laminar layer 151 defined by the plurality of pores 5 as illustrated in Figure 4.

According to an embodiment of the present invention, the main body 2 comprises at least one porous extremal layer 154 defined by a plurality of barrier pores 5' as illustrated in Figure 4.

Preferably, the extremal layer 154 is made in correspondence with an external face of the main body 2. In more detail, the extremal layer 154 is visible and/or facing outwards.

In other words, according to an embodiment of the present invention, the main body 2 is preferably made in a single body, i.e. preferably, the porous laminar layer 151 and the extremal layer 154 are obtained from the same silicon body 150 (in particular a monolithic body, e.g. without interruption).

In technical jargon of the sector, the main body 2 defines "a single double layer", i.e. provided with at least one porous laminar layer 151, defined by the plurality of pores 5, and at least one porous extremal layer 154, defined by the plurality of barrier pores 5'.

Preferably, the average diameter of the barrier pores 5' is smaller than the average diameter of the pores of said plurality of pores 5.

According to an embodiment of the present invention, each barrier pore 5' of said plurality of barrier pores 5' can have an average diameter preferably between 1 nm and 10 nm.

Preferably, each barrier pore 5' of said plurality of barrier pores 5' has a diameter equal to approximately 5 nm.

According to an embodiment of the present invention, said extremal layer 154 has a depth of 100 nm.

Obviously, the porous extremal layer 154 can have a depth greater or lesser than the aforementioned value, without thereby departing from the scope of protection of the present patent application.

Advantageously, the plurality of barrier pores 5' is configured to at least partially house the material of the support element 8.

More specifically, the average diameter of each barrier pore 5' is such as to allow partial housing of the material of the support element 8 while preventing said material of the support element 8 from passing through the entire depth of the porous extremal layer 154.

The average diameter of each barrier pore 5' and the depth of the extremal layer 154 contribute to the latter acting as a "barrier layer" against the penetration of the material (particularly polymeric) of the support element 8 during its heat welding, obviating the risk of decreasing the efficiency of the detection device 1 object of the invention.

The bonding surface 3 remains defined at least partially on each internal lateral wall of each pore 5.

In this way, the extension of the bonding surface 3 (made possible by the high porosity of the main body 2) is much greater than a planar, non-porous surface.

Advantageously, the extension of the binding surface 3 allows to amplify the number of drug molecules F that bind with the main body 2.

The main body 2 of the device 1 according to the invention allows to increase the intensity of the beam R1 of electromagnetic radiation emitted by the drug by about one hundred times compared to a layer of silicon oxide or of non-porous silica (i.e. full).

In fact, the porous structure defined by the plurality of pores 5 and by the extension of the binding surface 3 allows to increase the density of drug trapped inside the device 1.

With reference to the above amplification, the Applicant has conducted several tests and experiments, the results of which are summarized below.

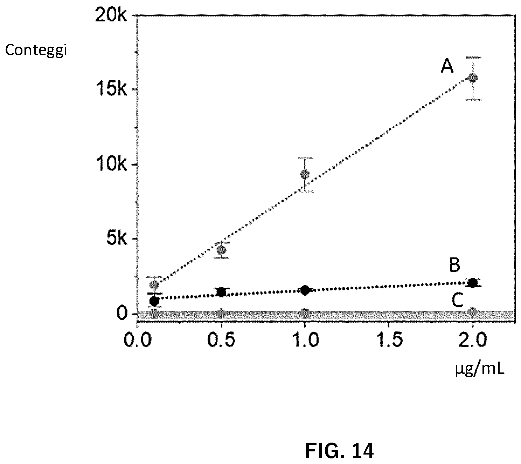

The attached Figure 14 shows a graphic in which the concentration of drug F, in particular doxorubicin, bound to the main body 2 of the device 1, is on the abscissa, while the ordinate shows the count (i.e. the number) of emissions of beams R1 of electromagnetic radiation emitted, in particular of photoluminescence emitted by three different specimens, according to curves A, B and C.

In more detail, the test counted the number of photons emitted at 590 nm wavelength, which corresponds to the peak of photoluminescence emission of doxorubicin. In this way, the test allows to detect the intensity of the beam R1 of electromagnetic radiation emitted by three different specimens, the result of which is illustrated by curves A, B and C.

In particular, the specimen whose behaviour is illustrated by curve C is formed by a full silicon oxide body, i.e. not provided with a porous structure.

It is evident that curve C is substantially horizontal, i.e. there is no emission of radiation at the peak at 590 nm. Therefore, it is not able to provide any information concerning the concentration of the drug F.

The specimen whose behaviour is illustrated by curve B is formed by a main silicon oxide body equipped with a porous structure, but not comprising any receptor 4.

It is evident that, although showing some counts of electromagnetic radiation corresponding to the emission peak at 590 nm (and therefore in fact at least a part of the drug F is actually emitting), such counts do not vary significantly with the variation of the concentration of drug that should be detected.

Therefore, even the specimen according to curve B is not optimal for the detection of the concentration of drug F.

The specimen whose behavior is illustrated by curve A is formed by a main body 1 according to the present invention, provided with the features described above.

It is immediately apparent that the counts of photoluminescence radiation in correspondence of the amplitude peak at 590 nm are detected and are directly proportional to the concentration of drug F.

Preferably, the photoluminescence radiation counts in correspondence of the amplitude peak at 590 nm are linearly dependent on the concentration of drug F.

Therefore, the tests performed by the Applicant confirm and demonstrate the desired technical effect, i.e. the direct link between the intensity of the beam R1 of electromagnetic radiation emitted by the device 1 and the concentration of drug F to be detected.

Operationally, the emission of the beam R1 of electromagnetic radiation by the drug F bound to the device 1 according to the invention can preferably be activated by an excitation beam R2 of electromagnetic radiation incident on the main body 2.

Advantageously, the wavelength of the excitation beam R2 of electromagnetic radiation is approximately equal to the intrinsic (or typical) absorption wavelength of the functional group of the drug F to be detected.

For example, in the case of doxorubicin, the wavelength of the excitation beam R2 of electromagnetic radiation is approximately equal to the absorption wavelength of the fluorophore group of doxorubicin, i.e. approximately equal to 480 nm.

The excitation beam R2 can be emitted by an external apparatus 100, as described in detail below.

The present invention also provides an assembly for detecting the concentration of a drug F, comprising at least one detection device 1 of the type described heretofore and of which the same numerical references will be maintained for simplicity of exposition.

All the technical features described with reference to the detection device 1 must be understood to be described - taken alone or in any combination thereof - also with reference to the detection assembly.

The assembly for detecting the concentration of a drug F comprising at least one device 1 according to one or more of the preceding claims and at least one external apparatus 100 configured to detect said electromagnetic radiation R1 emitted by said drug F and to convert said electromagnetic radiation R1 into a concentration value of said drug F.

Operationally, in use, the device 1 is intended to be implanted inside the body P of said patient and said external apparatus 100 is intended to be placed outside the body P of said patient, in proximity to said device 1.

Advantageously, in accordance with the preferential but non-limiting embodiment of the present invention illustrated in the attached Figures, said external apparatus 100 comprises at least one emitter module, sensor means and a processing module.

More specifically, the emitter module is configured to generate at least one excitation beam R2 of electromagnetic radiation, suited to intercept said detection device 1 and stimulate the emission of said beam R1 of electromagnetic radiation.

Conveniently, the excitation beam R2 hits the main body 1, exciting the drug F and forcing its functional group, in particular its fluorophore group, to emit the beam R1 in response.

In accordance with the preferred embodiment, the excitation beam R2 has a wavelength approximately equal to 480 nm, which preferably is equivalent to the absorption wavelength of the fluorophore group of doxorubicin, so that the beam R1 emitted by the main body 1 has an emission peak with a wavelength approximately equal to 590 nm.

Advantageously, the external apparatus 100 comprises sensor means, configured to receive said beam R1 of electromagnetic radiation emitted by said detection device 1 and generate an electronic signal corresponding to said intensity of said electromagnetic radiation.

For example, the sensor means may comprise an optical sensor, known per se to those skilled in the art, such as a photodiode, and therefore not described in detail below.

Advantageously, the external apparatus 100 comprises a processing module, configured to receive said electronic signal, process it, and generate at least one numerical value corresponding to said concentration of said drug F.

Preferably, the external apparatus 100 comprises at least one integrated circuit 102 on which are fixed and electronically connected at least the emitter module, the sensor means and the processing module.

Preferably, the external apparatus 100 comprises at least one data connection module, configured to send and/or receive electronic data from an external device, such as an external computer.

Preferably, the data connection module is configured to make a wireless connection with an external computer, for example via Bluetooth or Wi-Fi.

Preferably, the external apparatus 100 comprises fixing means 101 configured to mechanically couple the external apparatus 100 to the patient's body P at the area in which the detection device 1 has been inserted.

For example, the fixing means 101 may comprise a plaster or adhesive tape or even an adhesive material, preferably easily removable from the patient's body P.

Preferably, the device 1 is configured to detect the concentration of a drug F, such as in particular doxorubicin, continuously and in real time.

More preferably, the device 1 is configured to detect both an increase and a decrease in the concentration of the drug F.

Furthermore, the present invention also comprises a method of manufacturing a device for detecting a drug F, of the type described up to now and of which the same numerical references will be maintained for simplicity of exposition.

All the technical features described with reference to the detection device 1 must be understood as described - taken alone or in any combination thereof - also with reference to the manufacturing method.

The method in question provides for at least a preliminary step a) of preparing a silicon body 150.

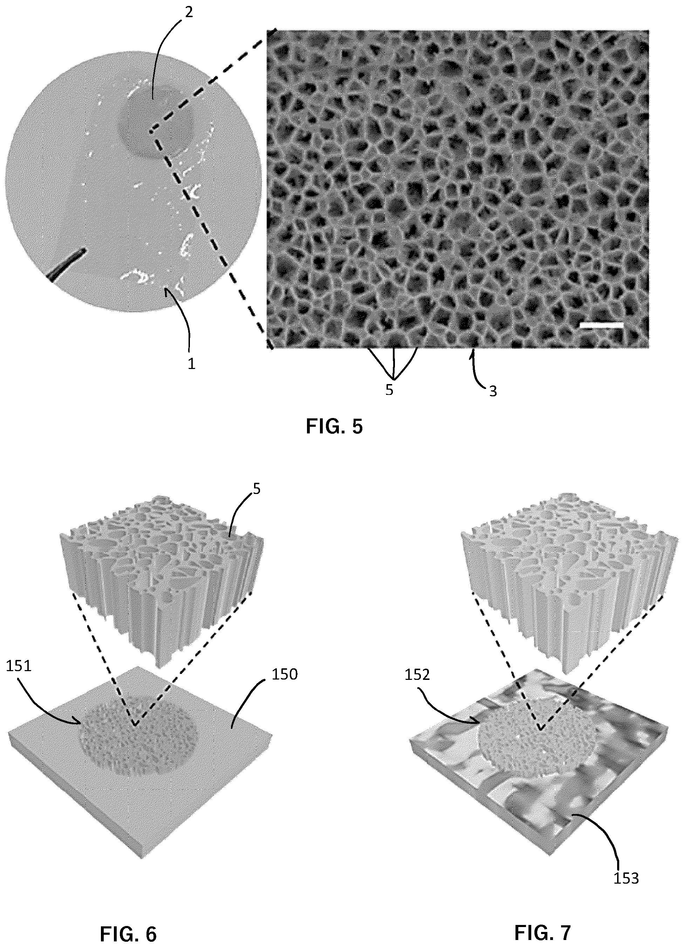

The method also comprises at least one step b) of manufacturing a plurality of pores 5 on the silicon body 150, to define a porous structure (illustrated in the attached Figures 5-6).

Preferably, step b) is performed by electrochemical etching.

Preferably, step b) involves creating the pores 5 on said silicon body 150 to define a porous laminar layer 151 with a depth preferably equal to approximately 5µm.

The method further comprises at least one step b') of making of a plurality of barrier pores 5' on the silicon body 150 (and/or on said porous laminar layer 151), to define a porous extremal layer (illustrated in the attached Figure 4).

Preferably, step b') is performed by electrochemical etching.

Preferably, step b') provides for realizing the barrier pores 5' on said silicon body 150 to define a porous extremal layer 154, in particular of depth preferably equal to approximately 100nm.

Preferably, following step b'), the porous laminar layer 151 comprises the extremal layer 154.

In other words, the porous laminar layer 151 and the porous extremal layer 154 are made in a monolithic structure, i.e. the main body 2 (comprising the layers 151 and 154) is made in a single body.

In the technical jargon of the sector, following the step b') a "single double layer" is obtained preferably comprising the porous laminar layer 151 and the porous extremal layer 154.

Preferably, the average diameter of the barrier pores 5' is smaller than the average diameter of the pores of said plurality of pores 5.

In accordance with a different embodiment, the steps a) - b') can be replaced by a step of preparing a porous laminar layer 151.

For example, the porous laminar layer 151 (preferably comprising the extremal layer 154) can be commercially available.

The method therefore comprises a step c) of removal of the porous laminar layer 151 from the silicon body 150 (not visible in the attached Figures).

The method therefore comprises a step d) of oxidation of the porous laminar layer 151 to obtain a layer of porous silicon oxide 152, in accordance with the attached Figure 7.

Preferably, the oxidation step d) comprises heating the porous laminar layer, in particular to a temperature of approximately 1000°C.

Advantageously, the step d) occurs by arranging the porous laminar layer 151 on a base 153 with high roughness.

For example, the porous laminar layer 151 can be silicon with high roughness, so that the porous laminar layer 151 does not melt together with the base 153.

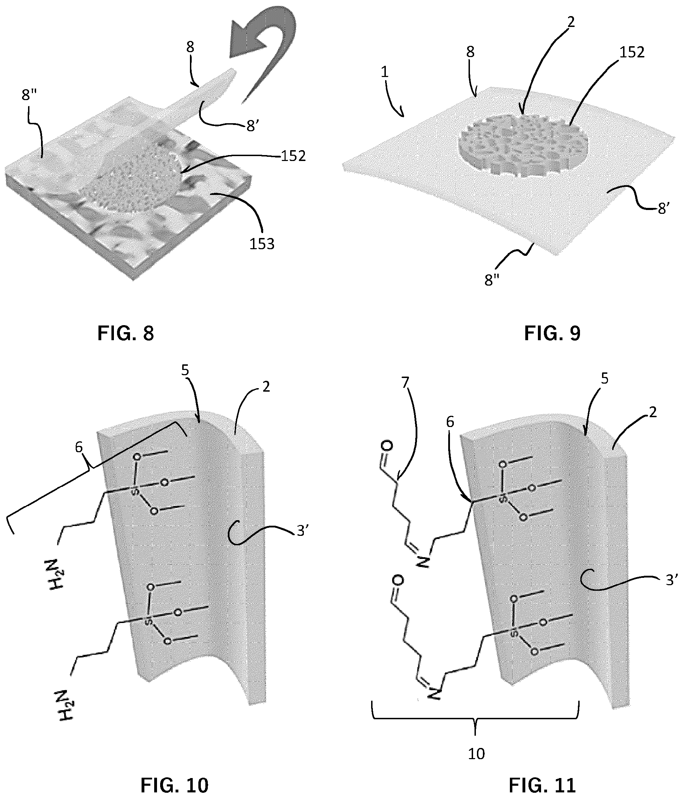

The method preferably comprises a step e) of fixing a support element 8 to the layer of porous silicon oxide 152 (illustrated in the attached Figures 8 and 9).

As described above, the support element 8 is made of polymeric material, in particular PLGA.

For example, step e) can be performed by heating the polymeric material of the support element 8 until it increases its viscosity and mechanically grips the porous structure of the layer of porous silicon oxide 152.

For example, step e) can involve heating the support element 8 to a temperature of approximately 50°C.

Subsequently, the method provides a step f) of treating the internal surface 3' of the pores 5 of the layer of porous silicon oxide 152.

More in detail, step f) provides for binding to the internal surface 3' at least one receptor 4 intended to chemically bind the drug F to be detected.

More in detail, step f) provides for a sub-step f1) of functionalizing the internal surface 3' of the plurality of pores 5 with at least one linking group 10.

More in detail, the linking group 10 of the sub-step f1) provides for binding to the silicon that makes up the layer of porous silicon oxide 152 at least one functionalizing agent 6 and, preferably, a coupling agent 7 (with particular reference to the attached Figures 10 and 11).

Preferably, step f) comprises a sub-step f2) of binding of the receptor 4 to the linking group 10 (see Figure 12).

Preferably, the linking group 10 comprises at least one functionalizing agent 6 and, preferably, at least one coupling agent 7.

The functionalizing agent 6 (and the coupling agent 7) is chemically interposed between the receptor 4 and the binding surface 3.

Advantageously, the internal surface 3' of each pore 5 is functionalized with the functionalizing agent 6 (and with the coupling agent 7) to allow chemical binding with the receptor 4.

Preferably, the functionalizing agent 6 is selected from the silane family. More preferably, the functionalizing agent 6 comprises an aminosilane and in particular (3-aminopropyl)triethoxysilane, known in jargon with the acronym APTES.

Advantageously, the coupling agent 7 chemically binds the functionalizing agent 6 to the receptor 4.

More specifically, the coupling agent 7 is chosen from the family of aliphatic dialdehydes and, in particular, comprises glutaraldehyde, known in jargon by the acronym GA.

In this way, following step f), the method according to the invention allows to obtain a detection device suited to detect the concentration of a drug, such as in particular doxorubicin.

Preferably, the method according to the invention allows to obtain a detection device suited to detect the concentration of a drug, such as in particular doxorubicin, continuously and in real time.

Therefore, the method according to the invention preferably allows to obtain a detection device suited to detect both an increase and a decrease in the concentration of a drug, such as in particular doxorubicin.

The present invention can be implemented in other variants all falling within the scope of the inventive features claimed and described; these technical features can be replaced by different technically equivalent elements and the materials used; the shapes and dimensions of the invention can be any as long as they are compatible with its use.

The numbers and reference signs inserted in the claims and in the description have the sole purpose of increasing the clarity of the text and must not be considered as elements that limit the technical interpretation of the objects or processes identified by them.

## Claims

1. Device (1) for detecting the concentration of a drug (F), wherein said drug (F) comprises at least one functional group suited to emit at least one beam (R1) of electromagnetic radiation, said device (1) comprises at least one main body (2) provided with a binding surface (3) intended to intercept said drug (F) to be detected; **characterized in that** said main body (2) is provided with a porous structure defined by a plurality of pores (5) delimiting within them said binding surface (3);
said main body (2) comprises at least one receptor (4) bound to said binding surface (3) and configured to chemically bind said drug (F) in said plurality of pores (5);
said intensity of said beam (R1) of electromagnetic radiation emitted by said drug (F) bound in said plurality of pores (5) being proportional to said concentration of said drug (F).

2. Device (1) for detecting the concentration of a drug (F) according to claim 1, **characterized in that** said main body (2) has a laminar shape and extends over a development plan (X).

3. Device (1) for detecting the concentration of a drug (F) according to one or more of the preceding claims, **characterized in that** said receptor (4) of said binding surface (3) comprises human serum albumin.

4. Device (1) for detecting the concentration of a drug (F) according to one or more of the preceding claims, **characterized in that** each pore (5) of said plurality of pores (5) has an average diameter between 10 and 50 nm.

5. Device (1) for detecting the concentration of a drug (F) according to one or more of the preceding claims, **characterized in that** said binding surface (3) is between 50 and 500 m²/cm³.

6. Device (1) for detecting the concentration of a drug (F) according to one or more of the preceding claims, **characterized in that** said main body (2) has porosity greater than or equal to 60%.

7. Device (1) for detecting the concentration of a drug (F) according to one or more of the preceding claims, **characterized in that** said main body (2) is intended to be implanted in the body (P) of a patient and is made of bioresorbable material.

8. Device (1) for detecting the concentration of a drug (F) according to claim 7, **characterized in that** said bioresorbable material has null intrinsic fluorescence.

9. Device (1) for detecting the concentration of a drug (F) according to one or more of the preceding claims, **characterized in that** each pore (5) of said plurality of pores (5) extends along a development axis (Y) substantially orthogonal to said development plan (X).

10. Device (1) for detecting the concentration of a drug (F) according to one or more of the preceding claims, **characterized in that** said receptor (4) is chemically bound to an internal surface (3') of each pore (5) of the main body (2) by means of at least one linking group (10).

11. Device (1) for detecting the concentration of a drug (F) according to one or more of the preceding claims, **characterized in that** said main body (2) comprises one or more porous laminar layers.

12. Device (1) for detecting the concentration of a drug (F) according to one or more of the preceding claims, **characterized in that** said main body (2) comprises at least one porous extremal layer (154) defined by a plurality of barrier pores (5').

13. Device (1) for detecting the concentration of a drug (F) according to one or more of the preceding claims, **characterized in that** the average diameter of said barrier pores (5') is smaller than the average diameter of the pores of said plurality of pores (5).

14. Assembly for detecting the concentration of a drug (F) comprising at least one device (1) according to one or more of the preceding claims and at least one external apparatus (100) configured to detect said electromagnetic radiation emitted by said drug (F) and to convert said electromagnetic radiation into a concentration value of said drug (F);
said device (1) being intended to be implanted inside the body (P) of said patient and said external apparatus (100) being intended to be placed outside the body (P) of said patient, in proximity to said device (1).

15. Assembly for detecting the concentration of a drug (F) according to claim 14, **characterized in that** said external apparatus (100) comprises:
- an emitter module, configured to generate at least one excitation beam (R2) of electromagnetic radiation, suited to intercept said detection device (1) and stimulate the emission of said beam (R1) of electromagnetic radiation;
- sensor means, configured to receive said beam (R1) the electromagnetic radiation emitted by said detection device (1) and generate an electronic signal corresponding to said intensity of said electromagnetic radiation;
- a processing module, configured to receive said electronic signal, process it, and generate at least one numerical value corresponding to said concentration of said drug (F).
